# FASCICULE DE BREVET EUROPEEN

(11) **EP 1 667 571 B1**
(45) Date de publication et mention de la délivrance du brevet: **25.03.2009**
(21) Numéro de dépôt: 04817092.2
(22) Date de dépôt: 29.09.2004
(51) Int. Cl.: A61B 1/005, A61B 1/31, G02B 23/24

(54) **FIBROSCOPE A TUBE D'INSERTION SEPARABLE**
FASERENDOSKOP MIT TRENNBAREM EINFÜHRROHR
FIBERSCOPE WITH A SEPARABLE INSERTION TUBE

(30) Priorité: 30.09.2003 FR 0311438
(43) Date de publication de la demande: 14.06.2006
(73) Titulaire: Dioptik SAS, 87069 Limoges Cedex (FR)
(72) Inventeur: Queyroux, Alain, 23000 Gueret (FR); Jouineau, Francois, 23000 Gueret (FR); Faugeras, Pierre, 87000 Limoges (FR)
(74) Mandataire: Myon, Gérard Jean-Pierre
(86) Numéro de dépôt international: PCT/FR2004/002465
(87) Numéro de publication internationale: WO 2005/032353

(56) Documents cités:
- US-A- 4 770 443
- US-A- 5 549 542
- US-A- 5 960 145

## Description

La présente invention concerne un fibroscope à tube d'insertion séparable.

Un fibroscope est un endoscope souple, permettant l'exploration des cavités profondes de l'organisme. A cet effet, ce fibroscope comprend un corps ainsi qu'un tube d'insertion, qui est destiné à pénétrer dans les cavités précitées.

Dans un passé récent, les performances des médecines pratiquant l'endoscopie se sont accrues grâce aux progrès technologiques, qui ont permis entre autres la réalisation d'endoscopes de plus en plus petits et, par voie de conséquence, de moins en moins traumatisants pour le patient.

En outre ces examens d'endoscopie, qui sont de plus en plus mis en oeuvre, deviennent indispensables à la pratique quotidienne. Ils permettent, d'une part, un diagnostic rapide et fiable et, d'autre part, le suivi régulier de nombreuses pathologies évitant ainsi, le plus souvent, d'avoir recours à d'autres séries d'examens volontiers plus coûteux ou plus agressifs.

Etant donné qu'ils font appel à un appareillage optique et électro-optique sophistiqué, les endoscopes présentent généralement un coût élevé de sorte que, pour des raisons de rentabilité, ils doivent être réutilisés chez un grand nombre de patients. Dans ces conditions, une telle réutilisation nécessite une stérilisation ou, au moins, une désinfection de l'endoscope, entre deux patients successifs.

Cependant, une telle stérilisation ne s'avère pas toujours totalement fiable, ce qui induit des risques de contamination des patients. En effet, les endoscopes ne résistent pas aux températures très élevées, de sorte que cette stérilisation est opérée par l'intermédiaire de procédures, dont la fiabilité ne peut être considérée comme absolue, en particulier à l'égard de micro-organismes émergents, tel celui de la maladie de Kreuzfeld Jacob.

De plus, une telle stérilisation est d'une mise en oeuvre relativement longue, ce qui implique l'intervention de personnel spécifique, ainsi que la mise à disposition de locaux et d'équipements de protection individuelle spécifiques. Par ailleurs, cette stérilisation s'accompagne de l'emploi de produits toxiques, dont la durée d'efficacité est limitée dans le temps et qui sont susceptibles d'être dangereux pour l'environnement.

En conclusion, de tels endoscopes se révèlent d'un coût élevé, de sorte que les examens de routine qui en tirent parti sont particulièrement onéreux.

Afin de remédier à ces inconvénients, on a proposé, par EP-A-0 813 384, un endoscope dont le tube d'insertion peut être séparé du corps. Ainsi, après chaque utilisation, ce tube est détaché et jeté, puis se trouve remplacé par un nouveau tube d'insertion.

Ceci permet de s'affranchir de la mise en oeuvre d'une opération de stérilisation. En effet, le tube souillé n'est plus réutilisé, alors que le corps de l'endoscope, qui se trouve à l'extérieur du patient, n'a pas besoin d'être stérilisé après chaque examen.

Ceci étant précisé, l'invention se propose de réaliser un fibroscope à tube d'insertion séparable, présentant une structure mécanique simple et un coût de fabrication relativement réduit, tout en tirant parti d'un raccord rapide et commode entre le corps et le tube d'insertion.

US 5,960,145 décrit un fibroscope tel que définie dans le préambule de la revendication 1.

A cet effet, elle a pour objet un fibroscope comprenant un corps et un tube d'insertion appartenant à une partie séparable par rapport au corps, ce corps et cette partie séparable étant reliés mécaniquement au niveau d'une zone de raccord, ce fibroscope comprenant également :
- des premiers moyens de guidage dont est pourvu le corps, en particulier un premier jeu de câbles, aptes à être actionnés par un organe de manoeuvre appartenant au corps, en particulier une poignée ;
- des seconds moyens de guidage dont est pourvue la partie séparable, en particulier un second jeu de câbles, aptes à mettre en mouvement le tube d'insertion ;
- des premiers moyens optiques dont est pourvu le corps, aptes à transmettre la lumière vers la zone de raccord et à ramener une image de cette zone de raccord vers une zone de visualisation par un praticien, telle qu'un oculaire ;
- des seconds moyens optiques dont est pourvue la partie séparable, aptes à transmettre la lumière de la zone de raccord vers une extrémité distale du tube d'insertion et à ramener une image de cette extrémité distale du tube d'insertion vers la zone de raccord et
- des premier et second organes de raccord mécanique et de transmission optique, qui sont solidarisés de façon amovible en service, chaque organe étant solidaire de moyens de guidage correspondants de manière à transmettre un mouvement imparti par les premiers moyens de guidage vers les seconds moyens de guidage, ces organes de raccord mécanique et de transmission optique étant également aptes à transmettre la lumière provenant des premiers moyens optiques en direction des seconds moyens optiques et à ramener une image provenant des seconds moyens optiques vers les premiers moyens optiques.

Selon d'autres caractéristiques de l'invention :
- la zone de raccord définit un logement possédant des parois intérieures en forme de portion de sphère, alors qu'au moins un parmi les premier et second organes de raccord mécanique et de transmission optique possède des parois extérieures sphériques de diamètre sensiblement égal à celui desdites parois intérieures, de façon à autoriser trois degrés de liberté en rotation, sans aucun degré de liberté en translation, de ces deux organes par rapport aux parois du logement.
- Les premier et second organes de raccord mécanique de transmission optique sont solidarisés de façon amovible, en service, en étant mutuellement fixés de façon amovible.
- Un premier organe de raccord mécanique et de transmission optique, pourvu desdites parois sphériques extérieures, définit une gorge de réception amovible d'un second organe de raccord mécanique et de transmission optique, qui est notamment un disque plan.
- Le premier organe de raccord mécanique et de transmission optique possède deux faces frontales parallèles ainsi qu'une couronne en saillie définissant, avec l'une de ces faces frontales, ladite gorge de réception.
- La zone de raccord comprend deux tronçons de raccord complémentaires, à peu près semi-cylindriques, appartenant respectivement au corps et à la partie séparable, tronçons de raccord dans lesquels sont ménagés des renfoncements correspondants, destinés à former en service ledit logement.
- Les premier et second organes de raccord mécanique et de transmission optique sont solidarisés de façon amovible en service, en étant coincés l'un par rapport à l'autre, notamment par plaquage mutuel.
- Les premiers moyens optiques comprennent une succession de lentilles associées à une source lumineuse.
- Les seconds moyens optiques comprennent un faisceau central de fibres optiques, aptes à ramener une image de l'extrémité distale du tube d'insertion vers la zone de raccord, ainsi qu'un faisceau périphérique de fibres optiques, aptes à transmettre la lumière de la zone de raccord vers cette extrémité distale.
- Le faisceau périphérique est entouré par une gaine, réalisée notamment en un matériau métallique ou plastique.
- Le faisceau central est formé de fibres optiques séparées.
- Le faisceau central est formé par différents faisceaux individuels de fibres optiques, de forme polyédrique, qui sont disposés les uns à côté des autres.
- La zone de raccord est entourée d'un moyen de verrouillage externe, en particulier une bague.

L'invention sera mieux comprise et d'autres avantages de celle-ci apparaîtront plus clairement à la lumière de la description qui va suivre d'un fibroscope conforme à son principe, donnée uniquement à titre d'exemple non limitatif et faite en référence aux dessins annexés, dans lesquels :
- la figure 1 est une vue de face, illustrant schématiquement un fibroscope conforme à l'invention ;
- la figure 2 est une vue en perspective, illustrant la zone de raccord entre le corps et le tube d'insertion du fibroscope de la figure 1 ;
- la figure 3 est une vue de côté, illustrant des éléments de transmission optique et de raccord mécanique, intervenant dans le fibroscope des figures 1 et 2 ; et
- la figure 4 est une vue en coupe longitudinale, illustrant la zone de raccord précitée, dans laquelle sont disposés les organes de transmission optique et de raccord mécanique de la figure 3 ;
- la figure 5 est une vue en coupe selon la ligne V-V à la figure 4.

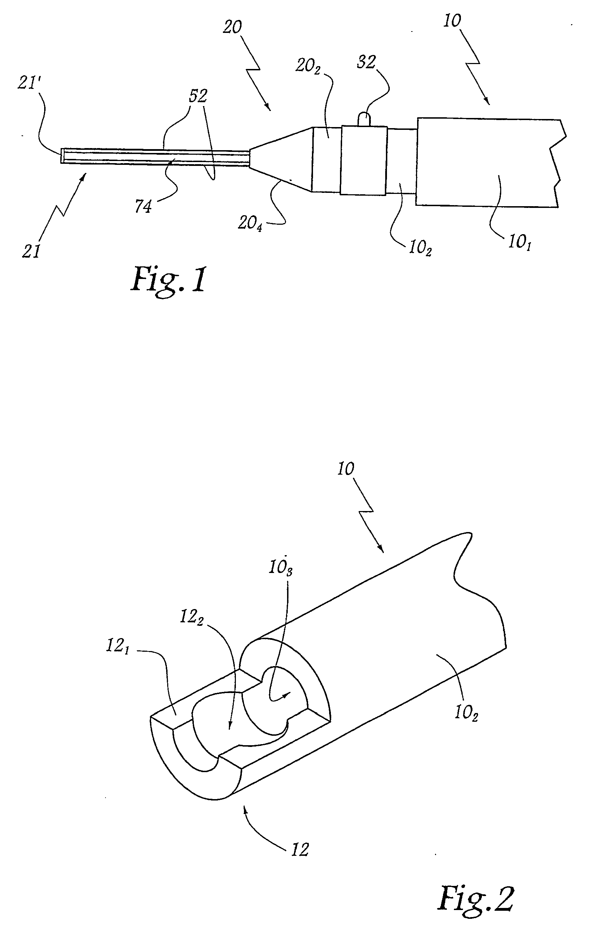

Comme le montre notamment la figure 1, le fibroscope de l'invention comprend un corps 10, destiné à la préhension par un utilisateur. Ce corps est pourvu d'une poignée, non représentée, permettant notamment de manoeuvrer des câbles de guidage, qui seront décrits plus en détail dans ce qui suit.

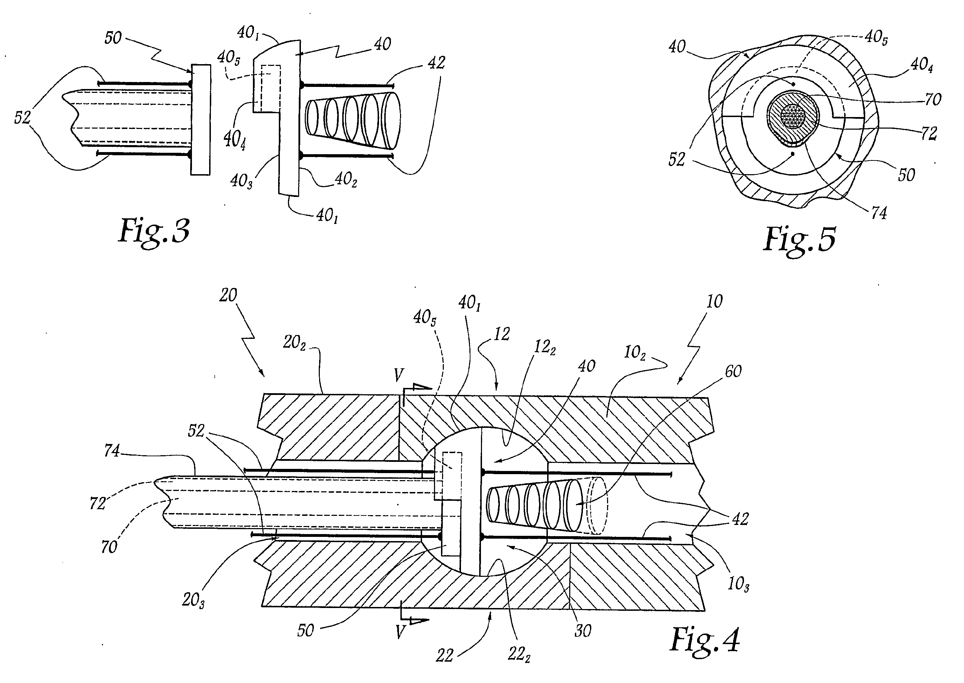

Le corps 10 comporte un manche 10₁, prolongé par un fût coaxial 10₂, de plus faible diamètre. Comme le montrent notamment les figures 2 et 5, un alésage central 10₃ est ménagé à l'intérieur de ce manche 10₁ et de ce fût 10₂.

Le fibroscope possède également une partie d'extrémité 20, séparable par rapport au corps 10, qui comprend un fût cylindrique 20₂, s'étendant dans le prolongement du fût 10₂ du corps 10. Ce fût 20₂ est lui-même prolongé par une région de liaison 20₄, de forme tronconique, qui est terminée par un tube d'insertion 21, destiné à pénétrer dans des cavités profondes du patient. Comme le montre la figure 5, le fût 20₂ est creusé d'un alésage central 20₃, coaxial à celui 10₃ ménagé dans le corps.

Ce corps 10 et cette partie séparable 20 se raccordent mécaniquement, au niveau d'une zone de raccord, qui est formée par deux tronçons de raccord 12 et 22, visibles sur la figure 5, appartenant respectivement au corps 10 et à la partie séparable 20. Sur la figure 2, seul est illustré le tronçon de raccord 12, étant entendu que la structure de celui 22 est analogue.

Comme le montre cette figure 2, le tronçon de raccord 12 affecte une forme globalement semi-cylindrique, formant ainsi un prolongement partiel du fût 10₂. Ce tronçon 12 est tronqué par un méplat diamétral 12₁, à partir duquel est creusé un renfoncement dont les parois 12₂ présentent une forme de portion de sphère. De façon analogue, le tronçon 22 est tronqué par un méplat, à partir duquel s'étend un renfoncement dont les parois 22₂ affectent également la forme d'une portion de sphère.

Ainsi, en service, les deux tronçons 12 et 22 sont en contact mutuel par leurs méplats respectifs, dont seul celui 12₁ est illustré. Par ailleurs, les deux renfoncements précités forment un logement 30, présentant des parois 12₂ et 22₂ sphériques, dans lequel débouchent les alésages 10₃ et 20₃. Cette zone de raccord 12, 22 est entourée par un moyen de verrouillage supplémentaire, qui est en l'occurrence une bague 32.

La figure 3 illustre des éléments de transmission optique et de raccord mécanique, équipant le fibroscope de l'invention. Il est ainsi prévu un disque transparent 40, formant lentille, dont la paroi extérieure 40₁ forme un tronçon de sphère, d'un diamètre correspondant à celui des parois 12₂ et 22₂ du logement 30.

Ce disque 40 est tronqué par une première face frontale 40₂, sur laquelle est fixé un premier jeu de câbles 42. Ces derniers, qui sont par exemple prévus au moins au nombre de deux, sont fixés sur le disque 40 par tout moyen approprié.

La seconde face frontale 40₃ du disque 40, parallèle à celle 40₂ évoquée ci-dessus, définit avec une couronne d'extrémité 40₄ une gorge 40₅. Cette dernière est destinée à la réception d'un disque transparent plat 50 de forme globalement circulaire. Ce disque 50 reçoit un second jeu de câbles 52, prévus au moins au nombre de deux, qui sont fixés par tout moyen approprié.

En service, comme le montre la figure 5, le disque 50 est inséré dans le volume intérieur de la gorge 40₅, délimitée par le disque 40. De la sorte, ces deux disques 40 et 50 sont mutuellement liés, à la fois en translation et en rotation.

Par conséquent, un déplacement des câbles 42, initié par la poignée, est transmis de façon correspondante aux câbles 52, par l'intermédiaire de ces deux disques 40 et 50. Les câbles 52 s'étendent jusqu'à l'extrémité distale du tube d'insertion 21, qui est formée par une lentille plate 21' (figure 1), où ils se trouvent fixés par tout moyen approprié.

On conçoit donc que la mise en mouvement précitée des câbles 42, qui induit une mise en mouvement correspondante des câbles 52, provoque un déplacement du tube d'insertion 21, sous la forme d'une torsion. En vue de faciliter un tel mouvement, la paroi extérieure de ce tube 21, qui est formée par une gaine comme on le verra dans ce qui suit, est avantageusement conformée à la manière d'un soufflet.

Le fibroscope de l'invention est muni, au niveau du corps 10, d'un système optique classique permettant, d'une part, d'envoyer de la lumière en direction du disque 40 et, d'autre part, de renvoyer une image provenant de ce dernier. Un tel système, qui est par exemple formé par une succession de lentilles, associées à une source lumineuse, est représenté de façon schématique sur la figure 4, où il se trouve affecté de la référence 60. A titre de variante, ce système optique peut également comprendre un arrangement de fibres optiques.

De façon analogue, la partie séparable 20 est pourvue de moyens permettant tout d'abord de transmettre, vers l'extrémité distale du tube d'insertion 21, la lumière provenant du système optique 60, via les disques 40 et 50. Ces moyens permettent également de ramener l'image, émise au niveau de cette extrémité distale du tube 21, en direction des disques 40 et 50 et, par conséquent, du système optique 60, de façon à en autoriser la visualisation par le praticien.

De façon plus précise, de tels moyens optiques comprennent deux faisceaux de fibres, à savoir tout d'abord un faisceau central cylindrique 70, apte à ramener l'image depuis l'extrémité distale du tube d'insertion 21. Ce faisceau 70 est entouré par un faisceau périphérique annulaire 72, destiné à envoyer la lumière en direction de cette extrémité distale.

Avantageusement, dans une configuration non représentée, le faisceau central 70 est formé par différents faisceaux individuels de fibres, de forme polyédrique, qui sont disposés les uns à côté des autres.

Comme le montrent notamment les figures 4 et 5, le faisceau périphérique 72 est entouré d'une gaine de protection 74, qui est par exemple de nature métallique ou plastique. Les faisceaux 70 et 72, associés à la gaine 74, se prolongent au-delà du fût 20₂ de la partie séparable 20, de manière à former le tube d'insertion 21, comme représenté à la figure 1. Au-delà de ce fût, comme le montre également cette figure 1, ces faisceaux 66 et 72, ainsi que cette gaine 74 sont entourés par les câbles 52, qui sont fixés sur la lentille 21' comme évoqué ci-dessus.

Après une utilisation du fibroscope de l'invention, il s'agit tout d'abord d'enlever la bague 32, puis de désolidariser la partie séparable 20 du corps 10, en dégageant le disque 50 de la gorge 40₅. Cette partie séparable, souillée au niveau du tube d'insertion 21, est alors jetée, puis remplacée par une autre partie séparable, de structure analogue, en vue d'une utilisation supplémentaire du fibroscope de l'invention.

L'invention n'est pas limitée à l'exemple décrit et représenté.

Ainsi, on peut prévoir de réaliser les disques 40, 50 de transmission optique et de raccord mécanique selon d'autres agencements. Par ailleurs, des canaux opératoires peuvent être adjoints aux différentes fibres optiques, en vue de l'aspiration, de la coagulation, ou encore du passage de pinces à biopsie ou d'autres instruments opératoires.

En service, les deux organes 40 et 50 peuvent être solidarisés de façon amovible, en étant fixés de manière amovible comme dans l'exemple illustré. A titre de variante, ils peuvent également être coincés l'un par rapport à l'autre, au sein de leur logement, notamment en étant plaqués l'un contre l'autre, de sorte qu'ils sont mutuellement liés à la fois en rotation et en translation, en service.

A titre de variante supplémentaire, au moins l'un des deux organes de raccord mécanique et de transmission optique peut être pourvu d'un pivot. Cette mesure permet de contraindre l'organe considéré à entrer en rotation, autour d'un unique axe.

A titre de variante supplémentaire, le faisceau central 70, permettant de ramener l'image de l'extrémité distale du tube d'insertion vers la zone de raccord, peut être formé par l'intermédiaire de fibres optiques séparées.

L'invention trouve son application à tous les domaines de l'endoscopie humaine ou animale. Dans le domaine médical, il s'agit notamment de l'oto-rhino-laryngologie, de l'endoscopie digestive oeso-gastro-duodénale et colique, de la bronchoscopie, de l'urologie ou encore de la gynécologie.

L'invention permet de réaliser les objectifs précédemment mentionnés.

Ainsi, le tube d'insertion, appartenant au fibroscope de l'invention, peut être mis en mouvement de manière fiable, grâce à la transmission des mouvements imprimés au niveau de la poignée du corps du fibroscope. Une telle transmission mécanique des mouvements est susceptible d'être mise en oeuvre dans toutes les directions, ce qui autorise un guidage précis du tube d'insertion.

De plus, la présence des disques 40 et 50, qui assurent une fonction supplémentaire de transmission optique, garantit la transmission de la lumière du corps vers l'extrémité du tube, ainsi que celle des images depuis cette extrémité en direction du corps.

En outre, le raccord mécanique, intervenant entre le corps et la partie séparable du fibroscope, est extrêmement simple et rapide à mettre en oeuvre. Ceci offre par conséquent une grande facilité d'utilisation et autorise l'emploi de tubes d'insertion stériles à usage unique. A cet égard, ces tubes d'insertion peuvent être reçus, après stérilisation par radiothérapie, dans des emballages eux aussi stériles, ce qui garantit une asepsie rigoureuse.

Il est également à noter que l'invention introduit une notion de traçabilité des différents tubes d'insertion. Ainsi, chaque tube est à même d'être affecté d'une référence spécifique.

Une fois utilisé, le tube d'insertion du fibroscope conforme à l'invention peut être éliminé de façon écologique, par exemple par incinération. Ceci permet d'éviter l'emploi de produits toxiques, notamment protéolytiques, qui ne sont pas retraités et se trouvent éliminés dans la nature.

L'invention permet enfin de s'affranchir des opérations classiques de décontamination. De la sorte, elle permet de réduire la main d'oeuvre d'une façon notable, et supprime sensiblement les taches dangereuses liées à la décontamination, prévalant dans l'art antérieur. L'invention permet également de réduire sensiblement le coût lié aux examens faisant appel à des fibroscopes.

## Revendications

1. Fibroscope comprenant un corps (10) et un tube d'insertion (21) appartenant à une partie (20) séparable par rapport au corps (10), ce corps (10) et cette partie séparable (20) étant reliés mécaniquement au niveau d'une zone de raccord (12, 22), ce fibroscope comprenant également :
- des premiers moyens de guidage dont est pourvu le corps (10), en particulier un premier jeu de câbles (42), aptes à être actionnés par un organe de manoeuvre appartenant au corps, en particulier une poignée ;
- des premiers moyens optiques (60) dont est pourvu le corps (10), aptes à transmettre la lumière vers la zone de raccord (12, 22) et à ramener une image de cette zone de raccord (12, 22) vers une zone de visualisation par un praticien, telle qu'un oculaire ;
- des seconds moyens optiques (70, 72) dont est pourvue la partie séparable (20), aptes à transmettre la lumière de la zone de raccord (12, 22) vers une extrémité distale (21') du tube d'insertion (21) et à ramener une image de cette extrémité distale du tube d'insertion (21) vers la zone de raccord (12, 22) et **caractérisé par**
- des seconds moyens de guidage dont est pourvue la partie séparable (20), en particulier un second jeu de câbles (52), aptes à mettre en mouvement le tube d'insertion (21) ; et par
- des premier (40) et second (50) organes de raccord mécanique et de transmission optique, qui sont solidarisés de façon amovible en service, chaque organe (40, 50) étant solidaire de moyens de guidage correspondants (42, 52) de manière à transmettre un mouvement imparti par les premiers moyens de guidage (42) vers les seconds moyens de guidage (52), ces organes (40, 50) de raccord mécanique et de transmission optique étant également aptes à transmettre la lumière provenant des premiers moyens optiques (60) en direction des seconds moyens optiques (70, 72) et à ramener une image provenant des seconds moyens optiques (70, 72) vers les premiers moyens optiques (60).

2. Fibroscope selon la revendication 1, **caractérisé en ce que** la zone de raccord (12, 22) définit un logement (30) possédant des parois intérieures (12₂, 22₂) en forme de portion de sphère, alors qu'au moins un parmi les premier (40) et second (50) organes de raccord mécanique et de transmission optique possède des parois extérieures (40₁) sphériques de diamètre sensiblement égal à celui desdites parois intérieures, de façon à autoriser trois degrés de liberté en rotation, sans aucun degré de liberté en translation, de ces deux organes par rapport aux parois du logement (30).

3. Fibroscope selon l'une quelconque des revendications 1 ou 2, **caractérisé en ce que** les premier (40) et second (50) organes de raccord mécanique de transmission optique sont solidarisés de façon amovible, en service, en étant mutuellement fixés de façon amovible.

4. Fibroscope selon les revendications 2 et 3, **caractérisé en ce qu'**un premier organe (40) de raccord mécanique et de transmission optique, pourvu desdites parois sphériques extérieures (40₁), définit une gorge (40₅) de réception amovible d'un second organe (50) de raccord mécanique et de transmission optique, qui est notamment un disque plan.

5. Fibroscope selon la revendication 4, **caractérisé en ce que** le premier organe (40) de raccord mécanique et de transmission optique possède deux faces frontales parallèles (40₂, 40₃) ainsi qu'une couronne (40₄) en saillie définissant, avec l'une (40₃) de ces faces frontales, ladite gorge de réception (40₅).

6. Fibroscope selon l'une quelconque des revendications 2 à 5, **caractérisé en ce que** la zone de raccord comprend deux tronçons de raccord (12, 22) complémentaires, à peu près semi-cylindriques, appartenant respectivement au corps (10) et à la partie séparable (20), tronçons de raccord dans lesquels sont ménagés des renfoncements correspondants, destinés à former en service ledit logement (30).

7. Fibroscope selon l'une des revendications 1 ou 2, **caractérisé en ce que** les premier et second organes de raccord mécanique et de transmission optique sont solidarisés de façon amovible en service, en étant coincés l'un par rapport à l'autre, notamment par plaquage mutuel.

8. Fibroscope selon l'une quelconque des revendications précédentes, **caractérisé en ce que** les premiers moyens optiques (60) comprennent une succession de lentilles associées à une source lumineuse.

9. Fibroscope selon l'une quelconque des revendications précédentes, **caractérisé en ce que** les seconds moyens optiques comprennent un faisceau central (70) de fibres optiques, aptes à ramener une image de l'extrémité distale du tube d'insertion (21) vers la zone de raccord (12, 22), ainsi qu'un faisceau périphérique (72) de fibres optiques, aptes à transmettre la lumière de la zone de raccord (12, 22) vers cette extrémité distale.

10. Fibroscope selon la revendication 9, **caractérisé en ce que** le faisceau périphérique (72) est entouré par une gaine (74), réalisée notamment en un matériau métallique ou plastique.

11. Fibroscope selon la revendication 9 ou 10, **caractérisé en ce que** le faisceau central (70) est formé de fibres optiques séparées.

12. Fibroscope selon la revendication 9 ou 10, **caractérisé en ce que** le faisceau central est formé par différents faisceaux individuels de fibres optiques, de forme polyédrique, qui sont disposés les uns à côté des autres.

13. Fibroscope selon l'une quelconque des revendications précédentes, **caractérisé en ce que** la zone de raccord (12, 22) est entouré d'un moyen de verrouillage externe, en particulier une bague (32).

## Claims

1. Fiberscope comprising a body (10) and an insertion tube (21) belonging to a part (20) that is separable from the body (10), this body (10) and this separable part (20) being mechanically joined at a connection zone (12, 22), this fiberscope also comprising:
- first guide means belonging to the body (10), in particular a first set of cables (42) that can be operated via a maneuvering element belonging to the body, in particular a handle;
- first optical means (60) belonging to the body (10) and able to transmit light to the connection zone (12, 22) and return an image of this connection zone (12, 22) to a zone for viewing by a practitioner, such as an eyepiece;
- second optical means (70, 72) belonging to the separable part (20) and able to transmit light from the connection zone (12, 22) to a distal end (21') of the insertion tube (21) and return an image from this distal end of the insertion tube (21) to the connection zone (12, 22), and **characterized by**
- second guide means belonging to the separable part (20), in particular a second set of cables (52) that are able to move the insertion tube (21) and by;
- first (40) and second (50) mechanical connection and optical transmission elements which are joined removably in service, each element (40, 50) being integral with corresponding guide means (42, 52) in such a way that a movement imparted by the first guide means (42) can be transmitted to the second guide means (52), these mechanical connection and optical transmission elements (40, 50) also being able to transmit light coming from the first optical means (60) to the second optical means (70, 72) and to return an image from the second optical means (70, 72) to the first optical means (60).

2. Fiberscope according to Claim 1, **characterized in that** the connection zone (12, 22) defines a seat (30) having inner walls (12₂, 22₂) in the shape of a portion of a sphere, while at least one of the first (40) and second (50) mechanical connection and optical transmission elements has spherical outer walls (40₁) with a diameter substantially equal to that of said inner walls, so as to allow three degrees of freedom in rotation, without any degree of freedom in translation, of these two elements relative to the walls of the seat (30).

3. Fiberscope according to either of Claims 1 and 2, **characterized in that** the first (40) and second (50) mechanical connection and optical transmission elements are joined removably, in service, by being mutually fixed in a removable manner.

4. Fiberscope according to either of Claims 2 and 3, **characterized in that** a first (40) mechanical connection and optical transmission element, provided with said outer spherical walls (40₁), defines a groove (40₅) for receiving, in a removable manner, a second (50) mechanical connection and optical transmission element, which is in particular a plane disk.

5. Fiberscope according to Claim 4, **characterized in that** the first (40) mechanical connection and optical transmission element has two parallel front faces (40₂, 40₃) and a protruding crown (40₄) defining, with one (40₃) of these front faces, said receiving groove (40₅).

6. Fiberscope according to any one of Claims 2 to 5, **characterized in that** the connection zone comprises two complementary, almost semicylindrical connection portions (12, 22) belonging respectively to the body (10) and to the separable part (20), in which connection portions corresponding recesses are formed which are intended to form said seat (30) in service.

7. Fiberscope according to either of Claims 1 and 2, **characterized in that** the first and second mechanical connection and optical transmission elements are joined removably in service by being wedged relative to one another, in particular by being pressed flat against one another.

8. Fiberscope according to any one of the preceding claims, **characterized in that** the first optical means (60) comprise a succession of lenses associated with a light source.

9. Fiberscope according to any one of the preceding claims, **characterized in that** the second optical means comprise a central bundle (70) of optical fibers that are able to return an image from the distal end of the insertion tube (21) to the connection zone (12, 22), and also a peripheral bundle (72) of optical fibers that are able to transmit light from the connection zone (12, 22) to this distal end.

10. Fiberscope according to Claim 9, **characterized in that** the peripheral bundle (72) is surrounded by a sheath (74), in particular made of a metal or plastic material.

11. Fiberscope according to Claim 9 or 10, **characterized in that** the central bundle (70) is made up of separate optical fibers.

12. Fiberscope according to Claim 9 or 10, **characterized in that** the central bundle is formed by different individual bundles of optical fibers of polyhedral shape which are disposed side by side one another.

13. Fiberscope according to any one of the preceding claims, **characterized in that** the connection zone (12, 22) is surrounded by an external locking means, in particular a ring (32)

## Patentansprüche

1. Fibroskop mit einem Körper (10) und mit einem Einführrohr (21), das zu einem vom Körper (10) trennbaren Teil (20) gehört, wobei dieser Körper (10) und dieser trennbare Teil (20) in Höhe eines Verbindungsbereichs (12, 22) mechanisch verbunden sind, wobei dieses Fibroskop ebenfalls aufweist:
- erste Führungseinrichtungen, mit denen der Körper (10) versehen ist, insbesondere ein erster Satz von Kabeln (42), die von einem zum Körper gehörenden Betätigungsorgan, insbesondere einem Handgriff, betätigt werden können;
- erste optische Einrichtungen (60), mit denen der Körper (10) versehen ist, die fähig sind, das Licht zum Verbindungsbereich (12, 22) zu übertragen und ein Bild dieses Verbindungsbereichs (12, 22) zu einem Anzeigebereich für einen Praktiker, wie einem Okular, zurückzuführen;
- zweite optische Einrichtungen (70, 72), mit denen der trennbare Teil (20) versehen ist, die fähig sind, das Licht vom Verbindungsbereich (12, 22) zu einem fernen Ende (21') des Einführrohrs (21) zu übertragen und ein Bild dieses fernen Endes des Einführrohrs (21) zum Verbindungsbereich (12, 22) zurückzuführen;
und **gekennzeichnet durch**
- zweite Führungseinrichtungen, mit denen der trennbare Teil (20) versehen ist, insbesondere einen zweiten Satz von Kabeln (52), die fähig sind, das Einführrohr (21) in Bewegung zu versetzen;
und **durch**
- erste (40) und zweite (50) Organe zur mechanischen Verbindung und zur optischen Übertragung, die im Betrieb lösbar miteinander verbunden sind, wobei jedes Organ (40, 50) fest mit entsprechenden Führungseinrichtungen (42, 52) verbunden ist, um eine von den ersten Führungseinrichtungen (42) verliehene Bewegung auf die zweiten Führungseinrichtungen (52) zu übertragen, wobei diese Organe (40, 50) zur mechanischen Verbindung und zur optischen Übertragung ebenfalls fähig sind, das von den ersten optischen Einrichtungen (60) kommende Licht in Richtung der zweiten optischen Einrichtungen (70, 72) zu übertragen und ein von den zweiten optischen Einrichtungen (70, 72) kommendes Bild zu den ersten optischen Einrichtungen (60) zurückzuführen.

2. Fibroskop nach Anspruch 1, **dadurch gekennzeichnet, dass** der Verbindungsbereich (12, 22) eine Aufnahme (30) definiert, die Innenwände (12₂, 22₂) in Form eines Kugelabschnitts besitzt, während mindestens eines der ersten (40) und zweiten (50) Organe zur mechanischen Verbindung und zur optischen Übertragung kugelförmige Außenwände (40₁) mit einem Durchmesser besitzt, der im Wesentlichen gleich demjenigen der Innenwände ist, um drei Rotations-Freiheitsgrade ohne jeden Translations-Freiheitsgrad dieser zwei Organe bezüglich der Wände der Aufnahme (30) zu erlauben.

3. Fibroskop nach einem der Ansprüche 1 oder 2, **dadurch gekennzeichnet, dass** das erste (40) und das zweite (50) Organ zur mechanischen Verbindung und zur optischen Übertragung im Betrieb lösbar miteinander verbunden sind, indem sie lösbar aneinander befestigt sind.

4. Fibroskop nach den Ansprüchen 2 und 3, **dadurch gekennzeichnet, dass** ein erstes Organ (40) zur mechanischen Verbindung und zur optischen Übertragung, das mit den kugelförmigen Außenwände (40₁) versehen ist, eine lösbare Aufnahmerille (40₅) für ein zweites Organ (50) zur mechanischen Verbindung und zur optischen Übertragung bildet, das insbesondere eine ebene Scheibe ist.

5. Fibroskop nach Anspruch 4, **dadurch gekennzeichnet, dass** das erste Organ (40) zur mechanischen Verbindung und zur optischen Übertragung zwei parallele Stirnseiten (40₂, 40₃) sowie einen vorstehenden Kranz (40₄) aufweist, der mit einer (40₃) dieser Stirnseiten die Aufnahmerille (40₅) definiert.

6. Fibroskop nach einem der Ansprüche 2 bis 5, **dadurch gekennzeichnet, dass** der Verbindungsbereich zwei komplementäre, in etwa halbzylindrische Verbindungsabschnitte (12, 22) aufweist, die zum Körper (10) bzw. zum trennbaren Teil (20) gehören, Verbindungsabschnitte, in denen entsprechende Vertiefungen ausgebildet sind, die dazu bestimmt sind, im Betrieb die Aufnahme (30) zu bilden.

7. Fibroskop nach einem der Ansprüche 1 oder 2, **dadurch gekennzeichnet, dass** das erste und das zweite Organ zur mechanischen Verbindung und zur optischen Übertragung im Betrieb lösbar miteinander verbunden sind, indem sie zueinander verkeilt sind, insbesondere durch gegenseitiges Andrücken.

8. Fibroskop nach einem der vorhergehenden Ansprüche, **dadurch gekennzeichnet, dass** die ersten optischen Einrichtungen (60) eine Folge von Linsen aufweisen, die einer Lichtquelle zugeordnet sind.

9. Fibroskop nach einem der vorhergehenden Ansprüche, **dadurch gekennzeichnet, dass** die zweiten optischen Einrichtungen ein zentrales Bündel (70) von Lichtleitfasern, die fähig sind, ein Bild des fernen Endes des Einführrohrs (21) zum Verbindungsbereich (12, 22) zurückzuführen, sowie ein Umfangsbündel (72) von Lichtleitfasern aufweisen, die fähig sind, das Licht vom Verbindungsbereich (12, 22) zu diesem fernen Ende zu übertragen.

10. Fibroskop nach Anspruch 9, **dadurch gekennzeichnet, dass** das Umfangsbündel (72) von einer Hülle (74) umgeben ist, die insbesondere aus einem Metall- oder Plastikwerkstoff hergestellt ist.

11. Fibroskop nach Anspruch 9 oder 10, **dadurch gekennzeichnet, dass** das zentrale Bündel (70) von getrennten Lichtleitfasern gebildet wird.

12. Fibroskop nach Anspruch 9 oder 10, **dadurch gekennzeichnet, dass** das zentrale Bündel von verschiedenen einzelnen polyederförmigen Lichtleitfaserbündeln gebildet wird, die nebeneinander angeordnet sind.

13. Fibroskop nach einem der vorhergehenden Ansprüche, **dadurch gekennzeichnet, dass** der Verbindungsbereich (12, 22) von einer äußeren Verriegelungseinrichtung umgeben ist, insbesondere von einem Ring (32).
